# EUROPEAN PATENT SPECIFICATION

(11) **EP 2 162 108 B1**
(45) Date of publication and mention of the grant of the patent: **09.12.2015**
(21) Application number: 08755431.7
(22) Date of filing: 14.05.2008
(51) Int. Cl.: A61F 13/58, A61F 13/60, A61F 13/62

(54) **FASTENING DEVICE**
BEFESTIGUNGSVORRICHTUNG
DISPOSITIF DE FIXATION

(30) Priority: 15.06.2007 US 944279 P
(43) Date of publication of application: 17.03.2010
(73) Proprietor: Avery Dennison Corporation, Pasadena, CA 91103 (US)
(72) Inventor: VAN STEEN, Johan, B-2380 Ravels (BE)
(74) Representative: Müller-Boré & Partner Patentanwälte PartG mbB
(86) International application number: PCT/US2008/063573
(87) International publication number: WO 2008/156931

(56) References cited:
- WO-A-98/22069
- JP-A- 10 137 008
- NL-A- 7 503 923
- US-A- 3 853 129
- US-A- 3 999 544
- US-A- 4 127 132
- US-A- 4 177 812
- US-A- 4 237 890
- US-A- 4 317 449
- US-A- 5 182 156
- US-A- 5 591 521
- US-A1- 2001 034 512
- US-A1- 2004 170 794
- US-A1- 2005 090 788
- US-A1- 2005 143 709
- US-B1- 6 451 000

## Description

### General Field

This application relates to a fastening device and, more particularly, to a fastening device having an anchor level, an extender level, and a fastener level hinged together for deployment in a fan-like fashion.

### Background

A fastening device can comprise an anchor level for permanent attachment to an anchoring surface, a fastener level for selective attachment to a fastening surface, and an extender level therebetween. A first hinge links a first end of the anchor level to a first end of extender level and a second hinge links a second end of the extender level to a second end of the fastener level. Prior to fastener deployment, it is usually preferred for the fastening device to have a flat profile wherein the levels compactly overlay each other. To deploy the fastening device, the fastener level is pivoted away from the extender level and the extender level is pivoted away from the anchor level to separate the layers in a fan-like fashion. The fastening device can be used, for example, on a diaper product for fitting and/or disposal purposes.

US 5 591 521 A discloses a fastening device with an anchor level comprising a first end section, a second end section, and an anchoring section therebetween for permanent attachment to an anchoring surface. The fastening device further comprises an extender level with a first end section, a second end section, and an extension section therebetween as well as a fastener level with a first end section, a second end section and a fastener section therebetween for selective attachment to a fastening surface. The fastening device further comprises a first hinge linking the anchor's first end section to the extender's first end section, a second hinge linking the extender's second end section to the fastener's second end section and a breakable joint joining respective sections of two adjacent levels prior to fastener deployment and breaking upon fastener deployment.

US 2004/170794 A1 discloses a U-folded fastener which is composed of different tape-like components which contain lines of weakening, which can be broken, upon which the fastener can be unfolded.

It is a problem to provide a breakable/weak connection between one or more different levels of the fastener without employing another component and the tape used for the fastener itself.

The problem is solved with the fastening device as described in claim 1. Preferred embodiments of this fastening device are contained in the subclaims.

### Summary

A fastening device has a breakable joint that joins respective sections of two adjacent levels prior to fastener deployment to hold or maintain the device in the preferred flat profile. The breakable joint is an integral joint; that is, it is an integral part of one of the levels and does not require the introduction of another component (e.g., a glue bead, a weld, a seam, etc.) during fastener fabrication. Specifically, the breakable joint is a designed weakness (e.g., perforations) in the relevant level. Upon fastener deployment, the breakable joint is broken, and the fastener level pivots relative to the extender level and the extender level pivots relative to the anchor level.

### Drawings

Figure 1 is a perspective view of a diaper including a pair of fastening devices.
Figure 2 is a perspective view of a diaper including a fastening device.
Figure 3 is a schematic side view of the fastening device in a predeployment condition.
Figures 3A - 3D are diagrams of the deployment process of the fastening device of Figure 3.
Figure 4 is a schematic side view of a fastening device which additionally includes a target tape.
Figures 4A - 4E are diagrams of the deployment process of the fastening device of Figure 4.
Figures 5 - 31 are schematic side views of modified versions of the fastening device.
Figures 32A - 32C are schematic side views of possible constructions for an extender level of the fastening device.
Figures 33A - 33C are schematic side views of possible constructions for a fastener level of the fastening device.

### Description

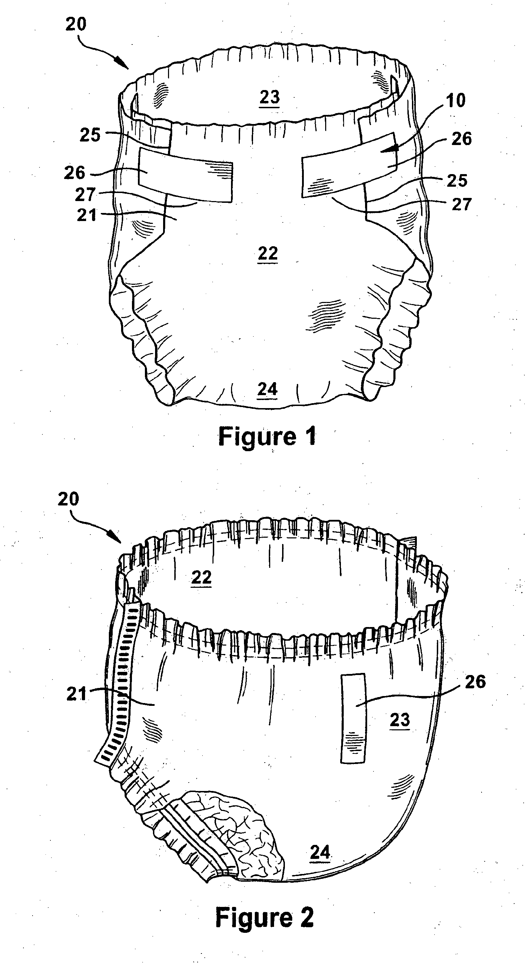

Referring now to the drawings, and initially to Figures 1 and 2, fastening devices 10 are shown installed on a diaper 20. The diaper 20 comprises a chassis 21 having a front portion 22, a rear portion 23, and a crotch portion 24 therebetween. In Figure 1, a pair of fastening devices 10 are used to secure the edges 25 of the rear portion 23 to the front portion 22 to thereby fit the diaper chassis 21 about the wearer. To this end, each fastening device 10 is anchored to an anchoring surface 26 (on the rear portion 23 and/or its edges 25) and selectively fastened to a fastening surface 27 (on the front portion 22). In Figure 2, the diaper 20 has a pants-like construction and the fastening device 10 is anchored to an anchoring surface 26 on the chassis rear portion 23. The fastening device 10 can be deployed and selectively fastened to a fastening surface (not specifically numbered) to secure a soiled diaper chassis 21 in a disposal configuration.

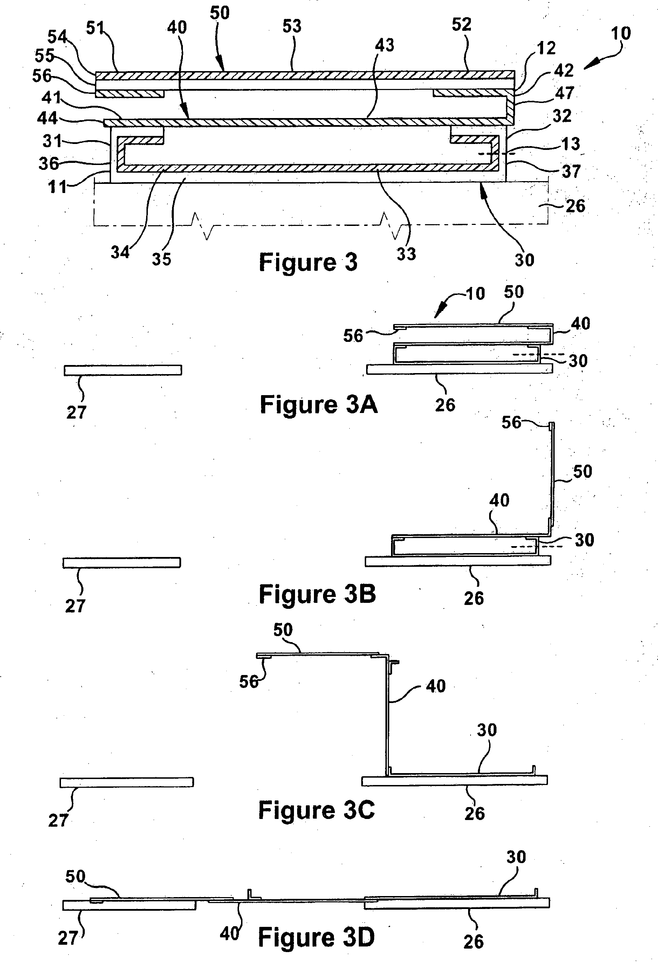

Referring now to Figure 3, the fastening device 10 is shown in a predeployment condition. The thicknesses of the levels/layers are greatly exaggerated in this and other drawings for ease of explanation. The thicknesses of these levels/layers will usually be in the range of, for example, about 2.5 micrometers to about 100 micrometers or more. If these thicknesses were drawn to scale with the illustrated lengths, it would be difficult to decipher and/or number the levels/layers. And neighboring levels/layers will generally be positioned flush against each other, even though the figures may give the impression that they are separated by gaps and do not contact one another.

The fastening device 10 comprises an anchor level 30, an extender level 40, and a fastener level 50. The anchor level 30 comprises first end section 31, a second end section 32, and an anchoring section 33 therebetween for permanent attachment to the anchoring surface 26. The extender level 40 comprises a first end section 41, a second end section 42, and an extension section 43 therebetween. The fastener level 50 comprises a first end section 51, a second end section 52, and a fastening section 53 therebetween for selective attachment to the fastening surface 27. In the illustrated orientation, the first end sections 31/41/51 are the left sections and the second end sections 32/42/52 are the right sections.

The anchor level 30 comprises a substrate 34 and an adhesive layer 35, the extender level 40 comprises a substrate 44 (without an adhesive layer), and the fastener level 50 comprises a substrate 54 and an adhesive layer 55.

The anchor level 30 has a fold 36 between its left section 31 and its anchoring section 33 and a fold 37 between its right section 32 and its anchoring section 33. The fold 36 and the fold 37 are each an inward and upward folds. This folded geometry results in the adhesive layer 35 facing the surface 26 in the anchoring section 33 and facing the end sections 41/42 of the extender level in the end sections 31/32. The adhesive layer 35 anchors the level 30 to the diaper surface 26, connects the anchor's left section 31 to the extender's left section 41, and connects the anchor's right section 32 to the extender level 40.

In the extender level 40, the left section 41 is not folded and is connected in a flat state (via the adhesive layer 35) to the anchor's left section 31. The extender level 40 has an inward and upward fold 47 between its right section 42 and its extension section 43. The fold 47 positions a rightmost region of the extension section 43 against the anchor's right section 32 (and the adhesive layer 35) and secures it thereto. And the fold 47 positions the right section 42 adjacent the right section 52 of the fastener section 50.

The fastener level 50 is not folded at either end section 51/52, and the adhesive layer 55 faces downward towards the extender level 40. The adhesive layer 55 connects the fastener's right section 52 to the folded right section 42 of the extender lever 40. The fastener level 50 can have a fingerlift 56 situated on its left section 51. In the illustrated embodiment, the fingerlift 56 is separate strip of material connected, via the adhesive layer 55, to the substrate 54. The fingerlift 56 could instead be a double substrate layer formed by folding over the distal end of the fastener level 50 (in which case it would be folded at its end section 51). Another option is for the fingerlift 56 to simply be an adhesive-free area.

The folded connection between the left sections 31/41 forms a hinge 11 linking the anchor level 30 to the extender level 40. The folded connection between the right sections 42/52 forms a hinge 12 linking the extender level 40 to the fastener level 50. In a predeployment condition,'the levels 30, 40, and 50 compactly overlay each other and the fastening device 10 has a generally flat profile. (Figure 3 and Figure 3A.) To deploy the fastening device 10, the fastener level 50 is pivoted away from the extender layer 40 by, for example, pulling the fingerlift 56 upward and to the right. (Figure 3B.) The extender level 40 is pivoted away from the anchor level 30 to separate the levels 30/40/50 in a fan-like fashion by, for example, pulling the fastener level 30 to the left. (Figure 3C.) As the fastener level 30 continues to move to the left, the levels 30/40/50 become aligned end-to-end and the fastener level 30 can be attached (via the adhesive layer 35) to the fastening surface 27. (Figure 3D.)

The folded connection between the anchor's right section 32 and the extender level 40 includes a breakable joint 13. As the extender level 40 is pulled away from the anchor level 30 during deployment, this joint 13 is broken to allow inter-level disconnect. (Compare Figure 3B and Figure 3C.) In the illustrated embodiment, the anchor's right section 32 is a "breakaway portion" which then remains with the extender level 40.

The breakable joint 13 reduces the risk of the extender level 40 prematurely pivoting away from the anchor level 30 prior to deployment. The breakable joint 13 is an integral joint, that is a joint that is integral with the relevant level itself. For example, the breakable joint 13 can be a designed weakness in the anchor level 30 and/or its substrate 34, such as series of perforations. Such an integral joint 13 does not require the introduction of another component (*e.g*., a glue bead, a weld, a seam; etc.) during fastener fabrication.

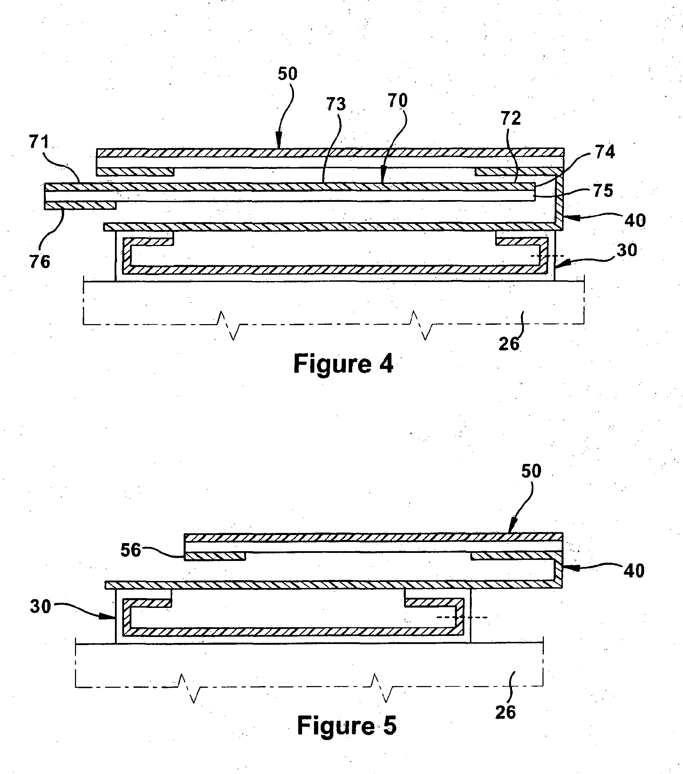

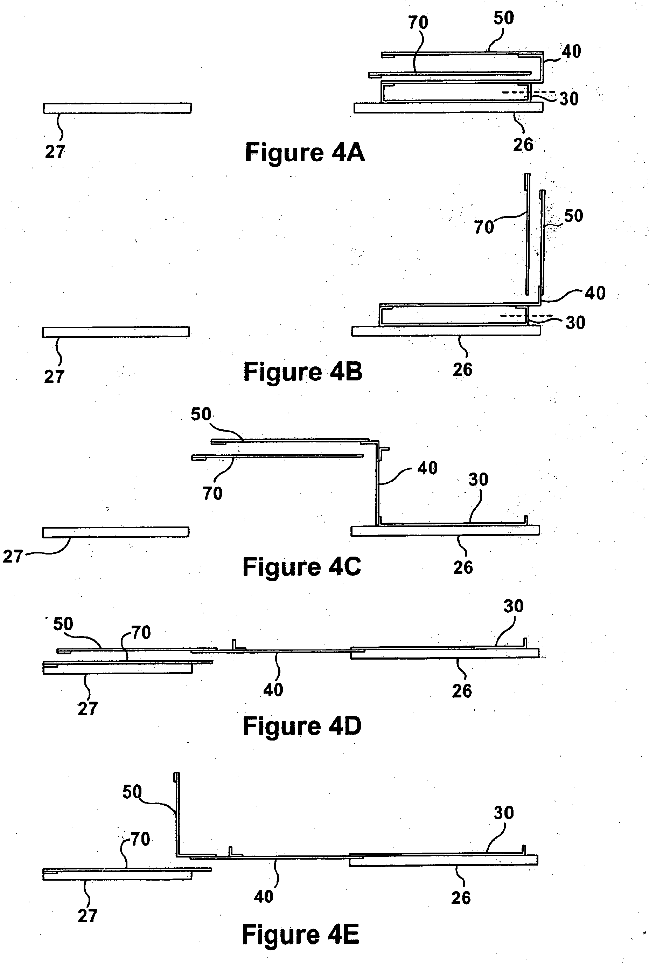

The fastener level 30 can attach directly to the fastening surface 27 or the fastening device 10 can include a target tape 70. (Figure 4.) The target tape 70 (which has a first end section 71, a second end section 72, and targeting section 73) can comprise a substrate 74 and an adhesive layer 75 on the lower side of the substrate 74 facing the extender level 40. The tape 70 can be positioned between the extender level 40 and the fastener level 50. The left section 71, which can include a fingerlift 76, can extend beyond the left of the fastener level 50. The tape's right section 72 can be positioned within the extender's righthand fold 47.

The target tape 70 is releasably attached to the fastener level 50 and carried therewith during fastener deployment. (Figures 4A - 4D.) Instead of pulling on the fastener fingerlift 56, the target-tape fingerlift 76 can be pulled to separate the level 50 from level 40 and to separate level 40 from level 30. At full deployment, the target tape 70 is attached to the fastening surface 27 with the fastener level 50 positioned thereover. (Figure 4D.) To open the fastening device 10 (e.g., to inspect the diaper 20), the fastener level 50 is pivoted away from the target tape 70 which remains attached to the surface 27. (Figure 4E.)

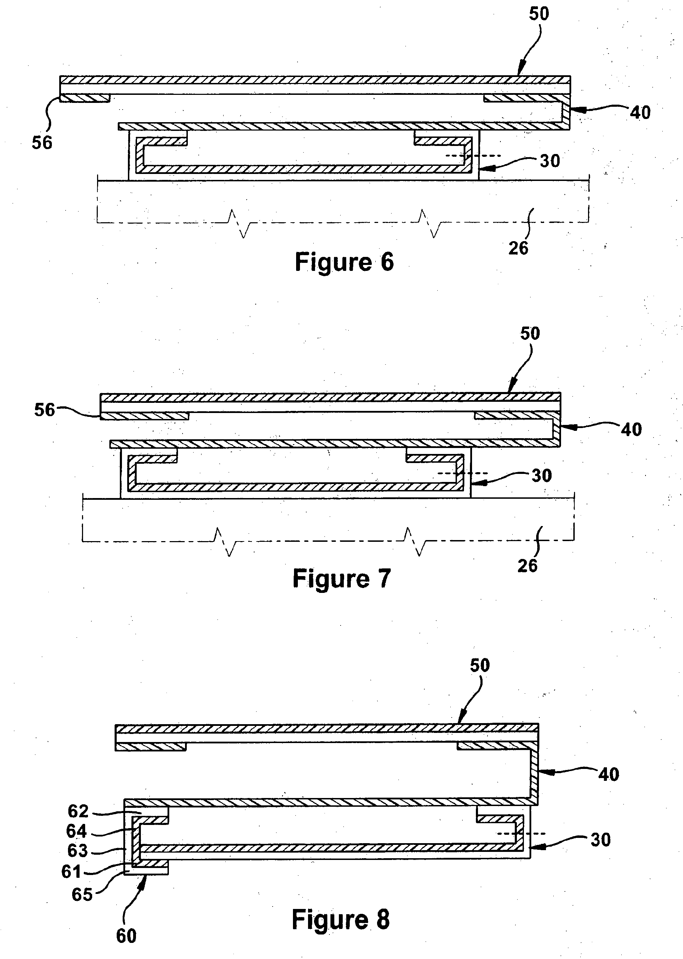

The anchor's left section 31 can be approximately aligned with the fastener's left section 51 and/or the anchor's right section 32 can be approximately aligned with the fastener's right section 52. (Figure 3.) Alternatively, the anchor's left section 31 can be offset from the fastener's left section 51 , either inwardly or outwardly. (Figures 5 and 6.) The anchor's right section 32 can be inwardly offset from the fastener's right section 52. (Figures 5 - 7.) Aligned-end-section arrangements can decrease false-fingerlift impressions, while offset-end-section arrangements can reduce maximum layer counts.

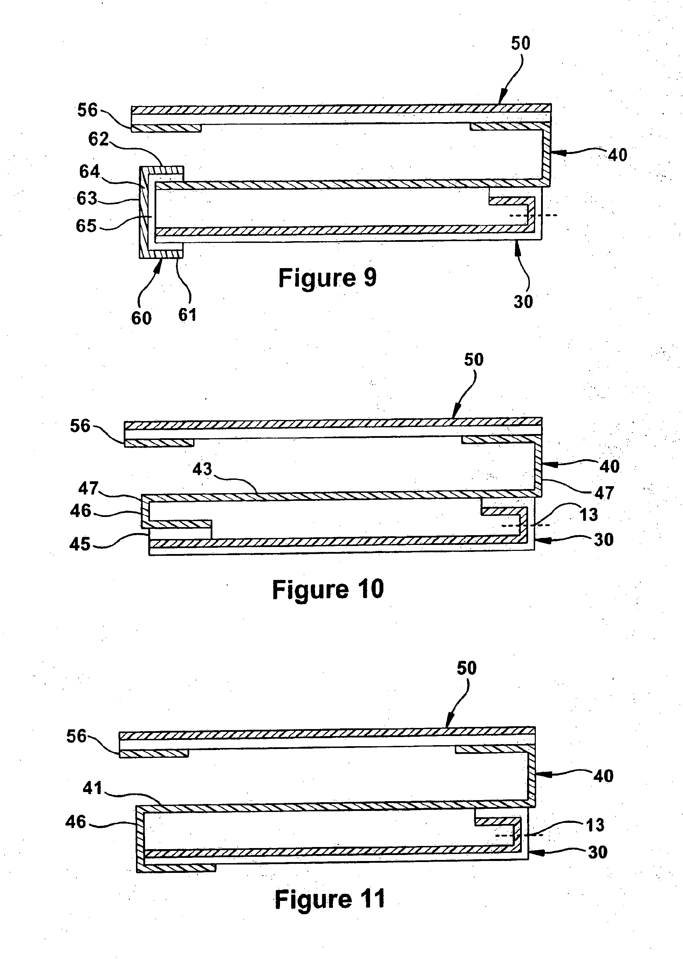

Instead of the lefthand hinge 11 being formed by the anchor's folded end section 31 , a separate strip 60 can be used. (Figures 8 and 9.) The illustrated strip 60 comprises end sections 61 and 62, and a section 63 completing its C-like or U-like shape. The end section 61 is connected to the anchor's left section 31 and the end section 62 is connected to the extender's left section 41. The strip 60 comprises a substrate 64 and an adhesive layer 65 on either the outer substrate side (Figure 8) or the inner substrate side (Figure 9).

The anchor's left folded end section 31 can also be replaced with a fold 46 in the extender level 40 between its left section 31 and its extension section 33. (Figures 10 and 11.) The fold 46, which is downward and inward, can be attached to the upper substrate face of the anchor's end section 31 via an adhesive layer 45 provided specifically for this purpose. (Figure 10.) Alternatively, the fold 46 can be attached to the lower adhesive face of the anchor's end section 31 , although this may give a false-fingerlift illusion. (Figure 11.)

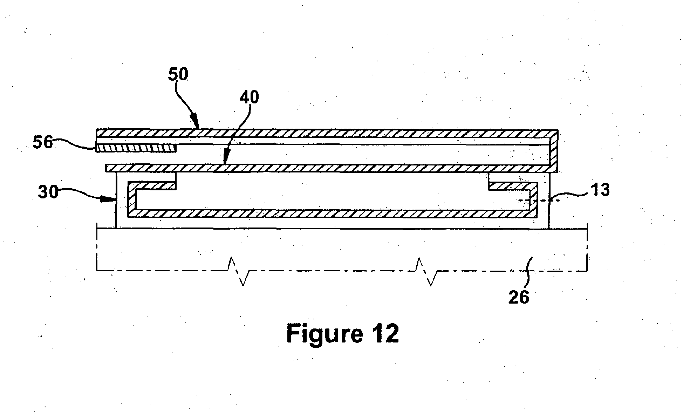

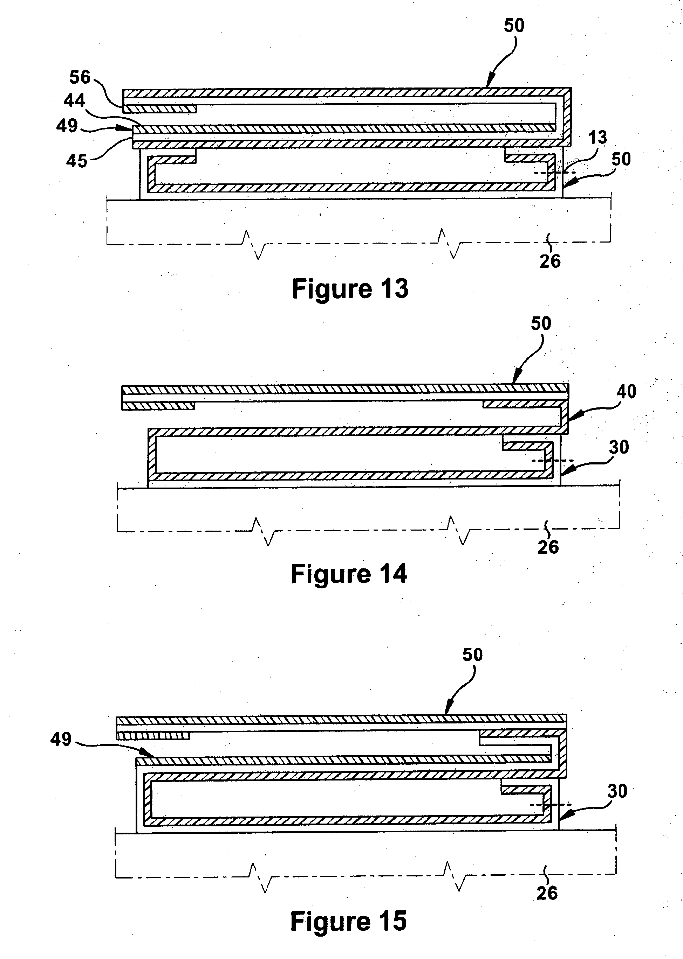

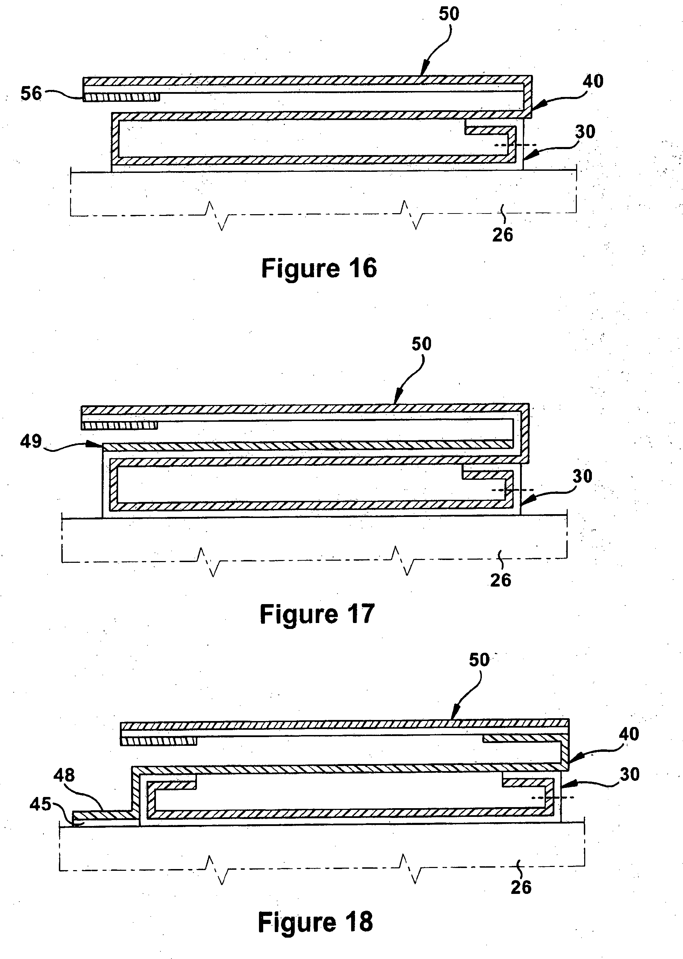

The levels 30/40/50 can be made from three separate tapes. Alternatively, the extender level 40 and the fastener level 50 can be made from a single tape (Figures 12 - 13), the anchor level 30 and the extender level 40 can be made from a single tape (Figures 14 - 15), or all three levels 30/40/50 can be made from a single tape (Figures 16 - 17). The multi-level tape can have a patterned adhesive layer 35/55 (Figures 12, 14, and 16) or it can have a continuous adhesive layer 35/45/55 covered with masking strip 49 in the extender level 40 (Figures 13, 15, and 17).

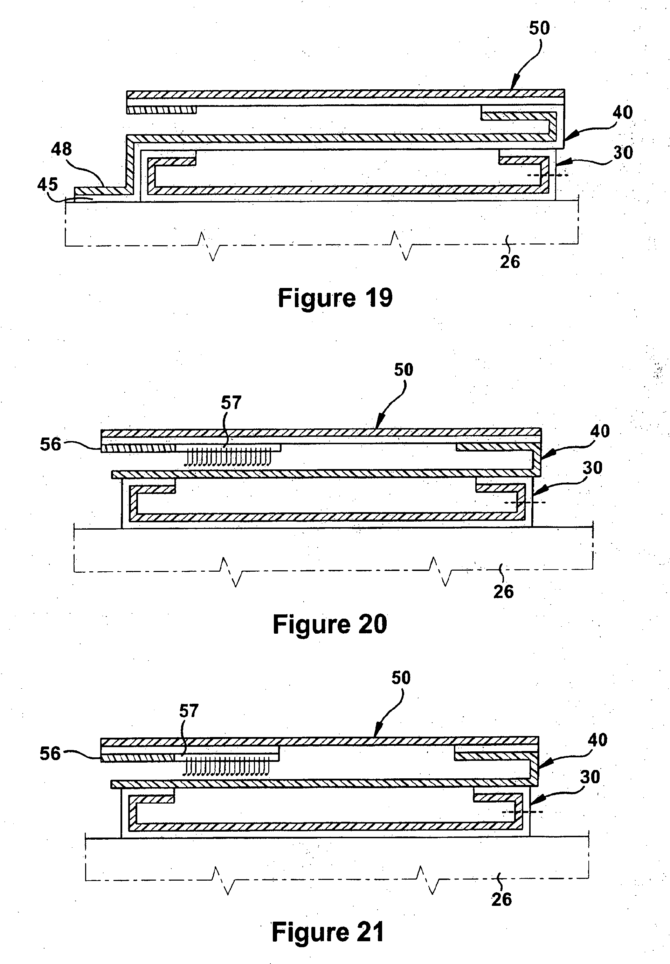

The extender level 40 can additionally comprise an anchor tail 48 trailing from its left section 41 and an adhesive layer 45 for attaching the tail 48 to the anchoring surface 26. (Figures 18 and 19.) The adhesive layer 45 can only occupy the tail portion of the extender level 40 (Figure 18) or it can occupy other portions as well (Figure 19).

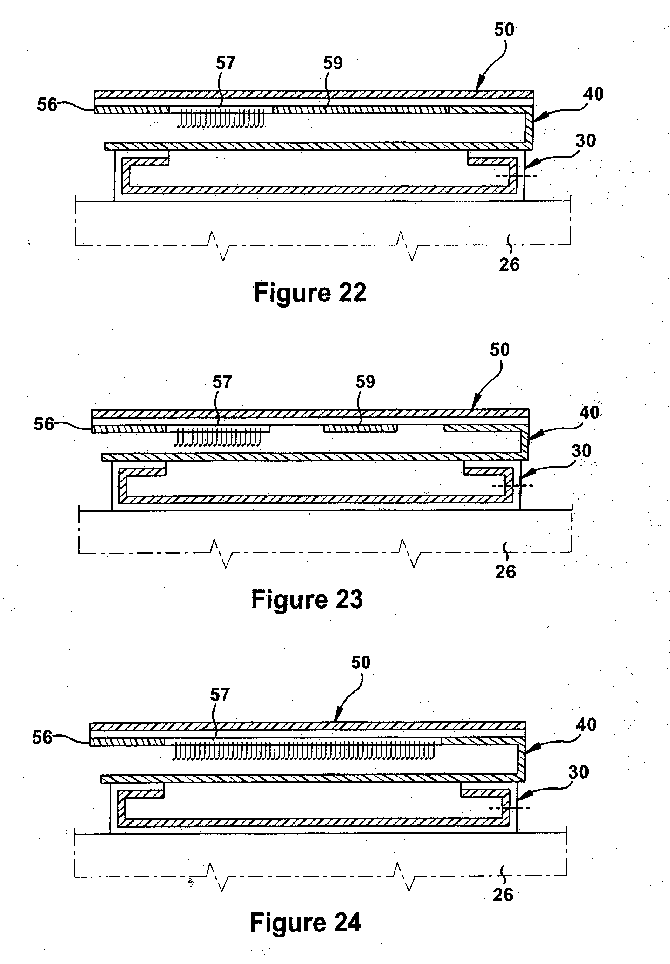

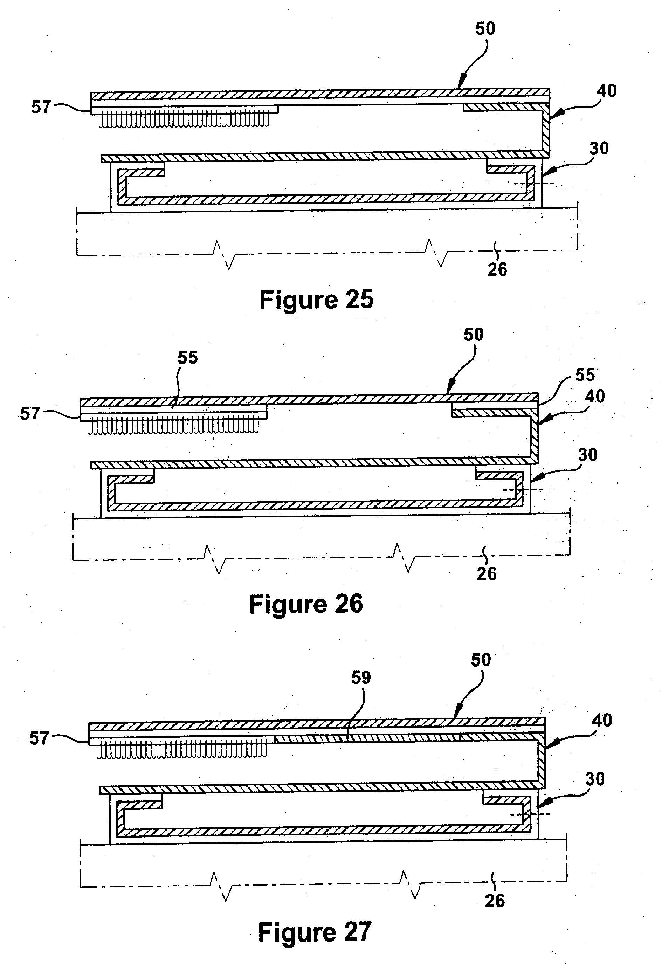

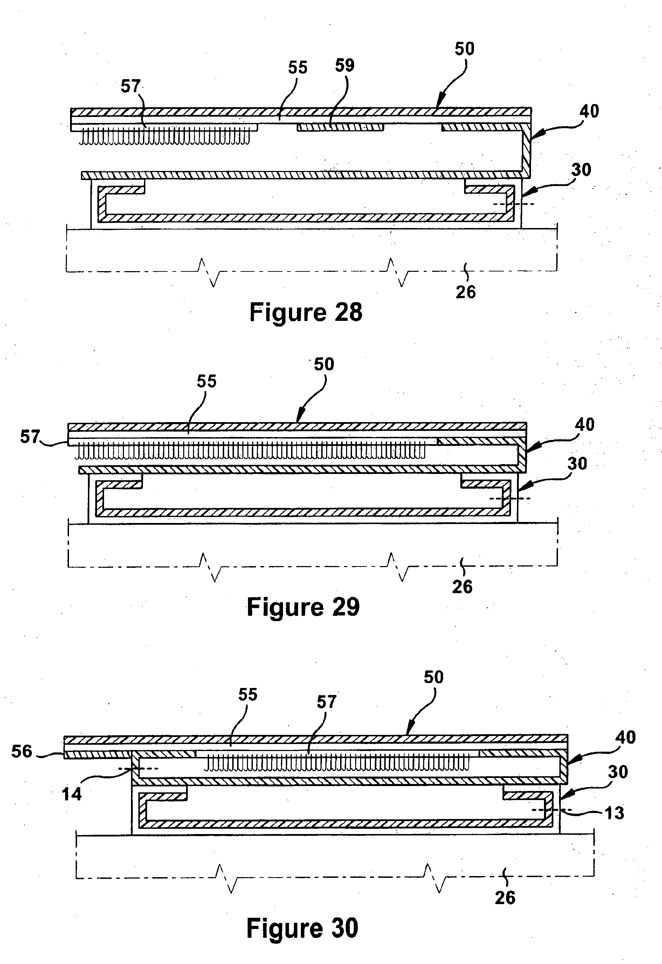

The fastener level 50 can attach to the fastening surface 27 via its adhesive layer 55. Additionally or alternatively, the fastener level 50 can comprise a patch 57 with hook members and the fastening surface 27 can comprise a complementary members. (Figures 20 - 29.) The hook patch 57 can be attached by the adhesive layer 55 to the substrate 54 and/or occupy at least a part of the fastening section 53 of the fastener level 50. The patch 57 can be used in conjunction with a fingerlift 56 (Figures 20 - 24) or can itself occupy the left section 51 of the fastener level 50 (Figures 25 - 29). The fastener level 50 can have exposed adhesive areas (Figures 20, 23, 25 and 28), covered adhesive areas (Figures 22, 23, 27 and 28), or adhesiveless areas (Figures 21 and 26) in the hook-unoccupied areas in the fastening section 33. The hook patch 57 can occupy the entire fastening section 33 with a fingerlift 56 (Figure 24) or without a fingerlift 56 (Figure 29).

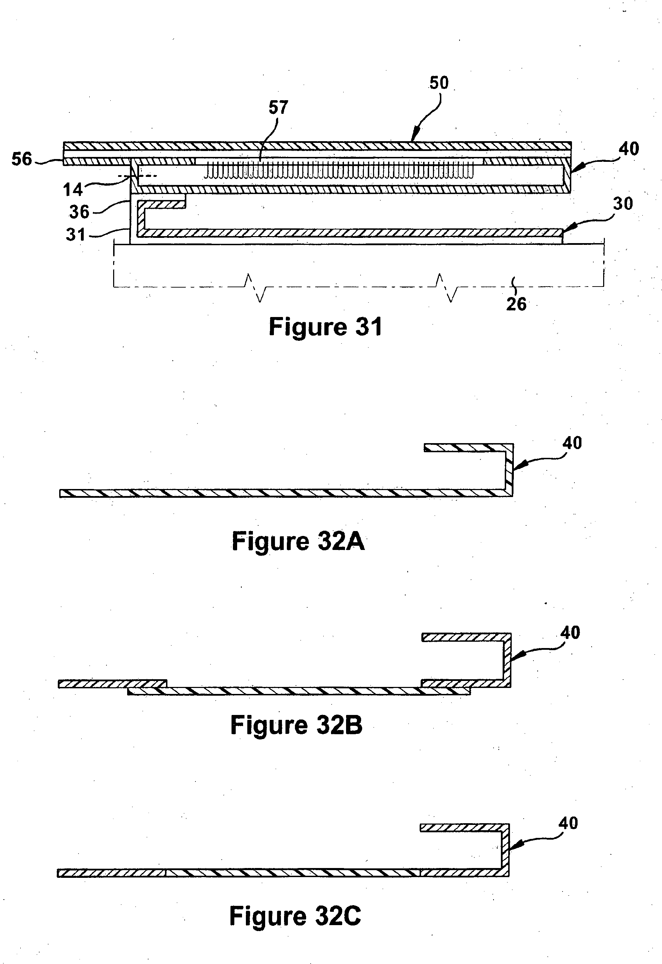

The fastening device 10 can additional or alternatively comprise an integral breakable joint 14 between its extender level 40 and its fastener level 50. (Figures 30 and 31.)

The substrates 34/44/54/74/84 can be made of cloth, kraft paper, cellophane film, non-woven webs, polymeric films (*e.g*., polypropylene, polyvinyl chloride, polyethylene terephthalate, and polyethylene) or other suitable materials or laminates. The non-adhesive sides of the substrates can include release coatings (*e.g*., a silicone coating, a carbamate coating, etc.) if such is necessary to prevent blocking issues during assembly, storing, and/or dispensing the fastening devices 10 for installation on the diaper 20.

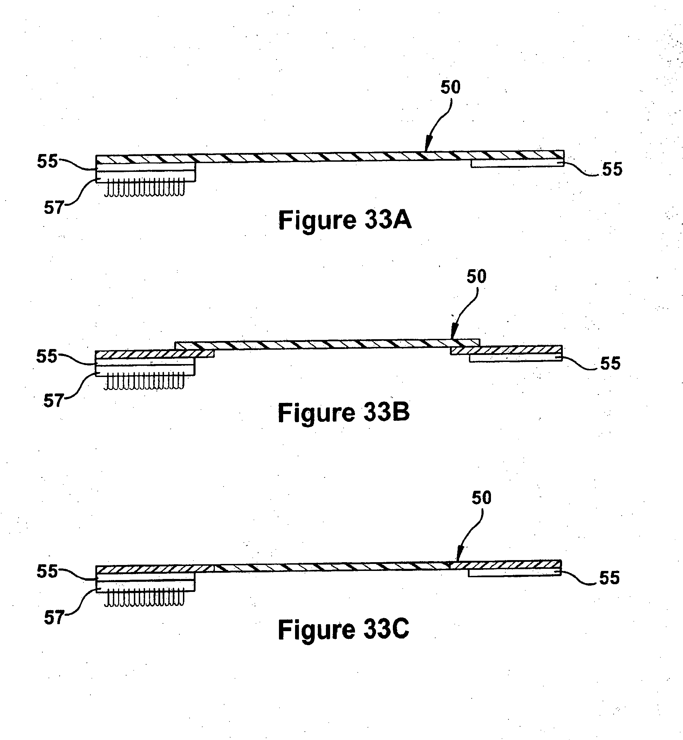

The anchor substrate 34, the extender substrate 44, and the fastener substrate 54 can have inelastic and/or elastic characteristics. The anchor substrate 34 will often be inelastic in view of its anchoring purposes. The extender substrate 44 and the fastener substrate 54 can be inelastic and, in many cases, this may be preferred. But an elastic extender level 40 can allow additional deployment length without a significant increase in the fastener's predeployment package size. To this end, the extender substrate 44 can be completely made of an elastic material (Figure 32A) or have elastic parts or regions (Figures 32B and 32C). The fastener substrate 54 can have similar elastic constructions. (Figures 33A - 33C.) The elastic substrates, parts, or regions can comprise elastic materials such as a SBL material, a NBL material, an elastomeric film, an elastomeric foam material, etc.

The adhesives layers 35/45/55/75/85 can comprise conventional adhesive, including pressure sensitive adhesives and non-pressure sensitive adhesives. Suitable pressure sensitive adhesives include acrylic resin and natural or synthetic based rubber adhesives. In many cases, the levels 30/40/50 can be constructed with continuous adhesive layers. "Continuous" in the present context refers to the fact that adhesive is not specifically applied to certain areas or zones of a substrate tape-segment/tape, but not others, according to the function of the area/zone in the fastener tab 10. Thus, a continuous layer can be, for example, a uninterrupted coating of adhesive or a regular/random coating pattern wherein the pattern is not dependent upon its application zone. That being said, patterned-coated adhesive layers may be preferred in certain situations.

The fingerlifts 56 and 76, and the masking strips 49 and 59, can be made of any suitable material. For example, they can be made of cloth, kraft paper, cellophane film, non-woven webs, polymeric films (*e.g*., polypropylene, polyvinyl chloride, polyethylene terephthalate, and polyethylene) and/or combinations thereof.

The hook patch 57 can comprise a base made of any material that is compatible with the hook-production process and accommodates its hook-carrying function. The actual hooks can be made of a plastic, metal or other material and formed by, for example, molding or stamping. The hooks can have a variety of "hooking" shapes such as, for example, a J-shape geometry, a mushroom-shape geometry, an arrow-shape geometry, a barbed geometry, a bulbous geometry.

The fastening device 10 has been described in conjunction with an absorbent article and it is well suited for diaper situations. But the fastening device 10 may also find use, in other applications. For example, the fastening device 10 could be used in packaging, safety garments, business items, and a variety of other disposable and nondisposable applications.

## Claims

1. A fastening device (10) comprising:
an anchor level (30) comprising a left first end section (31), a right second end section (32), and an anchoring section (33) therebetween for permanent attachment to an anchoring surface (26);
an extender level (40) comprising a left first end section (41), a right second end section (42), and an extension section (43) therebetween;
a fastener level (50) comprising a left first end section (51), a right second end section (52), and a fastening section (53) therebetween for selective attachment to a fastening surface (27);
a folded connection between the left first end sections (31, 41) forming a left first hinge (11) linking the anchor level's (30) left first end section (31) to the extender level's (40) left first end section (41);
a folded connection between the right second end sections (42, 52) forming a right second hinge (12) linking the extender level's (40) right second end section (42) to the fastener level's (50) right second end section (52); and
an integral breakable joint (13/14) joining respective sections of two adjacent levels prior to fastener deployment and breaking upon fastener deployment, wherein
the anchor level (30) comprises a right fold (37) between its anchoring section (33) and its right second end section (32), wherein
the integral breakable joint (13) is situated on the right fold (37) between the anchoring section (33) and the right second end section (32) and/or wherein
the extender level (40) comprises a left fold (47) between its extension section (43) and its left first end section (41), wherein
the integral breakable joint (14) is situated on the left fold (47) between the extension section (43) and the left first end section (41), wherein
the integral breakable joint (14) joins the extender level (40) to the fastener level (50) prior to fastener deployment.

2. A fastening device (10) as set forth in claim 1, wherein after fastener deployment, a break-away portion (32/41) of one adjacent level (30/40) remains attached to the other adjacent level (40/50).

3. A fastening device (10) as set forth in any of the preceding claims, wherein the breakable joint (13/14) consists of a series of perforations.

4. A fastening device (10) as set forth in any of the preceding claims, wherein the anchor level (30) comprises a substrate (34) and a layer of adhesive (35) on one side thereof.

5. A fastening device (10) as set forth in the preceding claim, wherein the layer of adhesive (35) attaches the anchoring section (33) to the anchoring surface (26).

6. A fastening device (10) as set forth in either claim 4 or claim 5, wherein the layer of adhesive (35) attaches the anchor level's right second end section (32) to the extender level's right second end section (42).

7. A fastening device (10) as set forth in any of claims 4 - 6 , wherein the layer of adhesive (35) attaches the anchor level's left first end section (31) to the extender level's left first end section (41).

8. A fastening device (10) as set forth in any of the preceding claims, wherein the extender level (40) additionally comprises an anchor tail (48) tailing from its left first end section (41) for attachment to the anchoring surface (26).

9. A fastening device (10) as set forth in any of the preceding claims, wherein the fastener level (50) comprises a substrate (54) and an adhesive layer (55) on the anchor level-adjacent side of the substrate (54).

10. A fastening device (10) as set forth in any of the preceding claims, further comprising a fingerlift (56) attached to the left first end section (51) of the fastener level (50).

11. A fastening device (10) as set forth in any of the preceding claims, further comprising a target tape (70) positioned between the extender level (40) and the fastener level (50), wherein the target tape (70) is preferably releasably attached to the fastener level (50) and carried therewith during fastener deployment.

12. A fastening device (10) according to any one of claims 1 to 10, further comprising a hook patch (57) attached to the anchor level-adjacent side of the fastener level (50).

13. A diaper (20) comprising a chassis (21) and at least one fastening device (10) as set forth in any of the preceding claims, the chassis (21) comprising an anchoring surface (26) and a fastening surface (27), the anchoring section (33) of the anchor level (30) being attached to the anchoring surface (26).

## Patentansprüche

1. Befestigungs- bzw. Verschlussvorrichtung (10), die umfasst:
eine Verankerungshöhe (30), die einen linken ersten Endabschnitt (31), einen rechten zweiten Endabschnitt (32) und einen Verankerungsabschnitt (33) dazwischen für die permanente Befestigung an einer Verankerungsfläche (26) umfasst;
eine Ansatzhöhe (40), die einen linken ersten Endabschnitt (41), einen rechten zweiten Endabschnitt (42) und einen Ansatzabschnitt (43) dazwischen umfasst;
eine Verschlusshöhe (50), die einen linken ersten Endabschnitt (51), einen rechten zweiten Endabschnitt (52) und einen Befestigungsabschnitt (53) dazwischen für die selektive Befestigung an einer Verschlussfläche (27) umfasst;
eine Faltverbindung zwischen den linken ersten Endabschnitten (31, 41), die ein linkes erstes Gelenk (11) bildet, das den linken ersten Endabschnitt (31) der Verankerungshöhe (30) mit dem linken ersten Endabschnitt (41) der Ansatzhöhe (40) verbindet;
eine Faltverbindung zwischen den rechten zweiten Endabschnitten (42, 52), die ein rechtes zweites Gelenk (12) bildet, das den rechten zweiten Endabschnitt (42) der Ansatzhöhe (40) mit dem rechten zweiten Endabschnitt (52) der Verschlusshöhe (50) verbindet; und
eine integrale zerreißbare Verbindung (13/14), die jeweilige Abschnitte von zwei benachbarten Höhen vor dem Einsatz des Verschlusses verbindet und nach dem Einsatz des Verschlusses zerreißt, wobei
die Verankerungshöhe (30) eine rechte Falte (37) zwischen ihrem Verankerungsabschnitt (33) und ihrem rechten zweiten Endabschnitt (32) umfasst, wobei
die integrale zerreißbare Verbindung (13) auf der rechten Falte (37) zwischen dem Verankerungsabschnitt (33) und dem rechten zweiten Endabschnitt (32) gelegen ist, und/oder wobei
die Ansatzhöhe (40) eine linke Falte (47) zwischen ihrem Ansatzabschnitt (43) und ihrem linken ersten Endabschnitt (41) umfasst, wobei
die integrale zerreißbare Verbindung (14) auf der linken Falte (47) zwischen dem Ausdehnungsabschnitt (43) und dem linken ersten Endabschnitt (41) gelegen ist, wobei
die integrale zerreißbare Verbindung (14) die Ansatzhöhe (40) vordem Verschlusseinsatz mit der Verschlusshöhe (50) verbindet

2. Verschlussvorrichtung (10) nach Anspruch 1, wobei ein Abrissabschnitt (32/41) einer benachbarten Höhe (30/40) an der anderen benachbarten Höhe (40/50) befestigt bleibt.

3. Verschlussvorrichtung (10) nach einem der vorhergehenden Ansprüche, wobei die zerreißbare Verbindung (13/14) aus einer Reihe von Perforationen besteht.

4. Verschlussvorrichtung (10) nach einem der vorhergehenden Ansprüche, wobei die Verankerungshöhe (30) ein Substrat (34) und eine Klebstoffschicht (35) auf einer seiner Seiten umfasst.

5. Verschlussvorrichtung (10) nach dem vorhergehenden Anspruch, wobei die Klebstoffschicht (35) den Verankerungsabschnitt (33) an der Verankerungsfläche (26) befestigt.

6. Verschlussvorrichtung (10) nach Anspruch 4 oder Anspruch 5, wobei die Klebstoffsicht (35) den rechten zweiten Endabschnitt (32) der Verankerungshöhe an dem rechten zweiten Endabschnitt (42) der Ansatzhöhe befestigt.

7. Verschlussvorrichtung (10) nach einem der Ansprüche 4 - 6, wobei die Klebstoffschicht (35) den linken ersten Endabschnitt (31) an dem linken ersten Endabschnitt (41) der Ansatzhöhe befestigt.

8. Verschlussvorrichtung (10) nach einem der vorhergehenden Ansprüche, wobei die Ansatzhöhe (40) zusätzlich einen Verankerungsausläufer (48) umfasst, der für die Befestigung an der Verankerungsfläche (26) von ihrem linken ersten Endabschnitt (14) ausläuft.

9. Verschlussvorrichtung (10) nach einem der vorhergehenden Ansprüche, wobei die Verschlusshöhe (50) ein Substrat (54) und eine Klebstoffschicht (55) auf der zu der Verankerungshöhe benachbarten Seite des Substrats umfasst.

10. Verschlussvorrichtung (10) nach einem der vorhergehenden Ansprüche, die ferner einen Fingerlift (56) umfasst, der an dem linken ersten Endabschnitt (51) der Verschlusshöhe (50) befestigt ist.

11. Verschlussvorrichtung (10) nach einem der vorhergehenden Ansprüche, die ferner ein Zielband (70) umfasst, das zwischen der Ansatzhöhe (40) und der Verschlusshöhe (50) positioniert ist, wobei das Zielband (70) vorzugsweise lösbar an der Verschlusshöhe (50) befestigt ist und während des Verschlusseinsatzes damit getragen wird.

12. Verschlussvorrichtung (10) nach einem der Ansprüche 1 bis 10, die ferner einen Hakenflecken (57) umfasst, der an der zu der Verankerungshöhe benachbarten Seite der Verschlusshöhe (50) befestigt ist.

13. Windel (20), die einen Körper (21) und wenigstens eine Verschlussvorrichtung (10) nach einem der vorhergehenden Ansprüche umfasst, wobei der Körper (21) eine Verankerungsfläche (26) und eine Verschlussfläche (27) umfasst, wobei der Verankerungsabschnitt (33) der Verankerungshöhe (30) an der Verankerungsfläche (26) befestigt ist.

## Revendications

1. Dispositif de fixation (10) comprenant :
un niveau d'ancrage (30) comprenant une première section d'extrémité gauche (31), une seconde section d'extrémité droite (32) et une section d'ancrage (33) entre elles pour une liaison permanente à une surface d'ancrage (26) ;
un niveau d'extension (40) comprenant une première section d'extrémité gauche (41), une seconde section d'extrémité droite (42) et une section d'extension (43) entre elles ;
un niveau de fixation (50) comprenant une première section d'extrémité gauche (51), une seconde section d'extrémité droite (52) et une section de fixation (53) entre elles pour une liaison sélective à une surface de fixation (27) ;
une connexion pliée entre les premières sections d'extrémité gauches (31, 41) formant une première charnière gauche (11) reliant la première section d'extrémité gauche (31) du niveau d'ancrage (30) à la première section d'extrémité gauche (41) du niveau d'extension (40) ;
une connexion pliée entre les secondes sections d'extrémité droites (42, 52) formant une seconde charnière droite (12) reliant la seconde section d'extrémité droite (42) du niveau d'extension (40) à la seconde section d'extrémité droite (52) du niveau de fixation (50) ; et
un joint cassant intégral (13/14) joignant des sections respectives de deux niveaux adjacents avant le déploiement de la fixation et cassant suite au déploiement de la fixation, dans lequel
le niveau d'ancrage (30) comprend un pli droit (37) entre sa section d'ancrage (33) et sa seconde section d'extrémité droite (32), dans lequel
le joint cassant intégral (13) est situé sur le pli droit (37) entre la section d'ancrage (33) et la seconde section d'extrémité droite (32) et/ou dans lequel
le niveau d'extension (40) comprend un pli gauche (47) entre sa section d'extension (43) et sa première section d'extrémité gauche (41), dans lequel
le joint cassant intégral (14) est situé sur le pli gauche (47) entre la section d'extension (43) et la première section d'extrémité gauche (41), dans lequel
le joint cassant intégral (14) joint le niveau d'extension (40) au niveau de fixation (50) avant le déploiement de la fixation.

2. Dispositif de fixation (10) selon la revendication 1, dans lequel, après le déploiement de la fixation, une portion de détachement (32/41) d'un niveau adjacent (30/40) reste liée à l'autre niveau adjacent (40/50).

3. Dispositif de fixation (10) selon l'une quelconque des revendications précédentes, dans lequel le joint cassant (13/14) est constitué d'une série de perforations.

4. Dispositif de fixation (10) selon l'une quelconque des revendications précédentes, dans lequel le niveau d'ancrage (30) comprend un substrat (34) et une couche d'adhésif (35) sur un côté de celui-ci.

5. Dispositif de fixation (10) selon l'une quelconque des revendications précédentes, dans lequel la couche d'adhésif (35) lie la section d'ancrage (33) à la surface d'ancrage (26).

6. Dispositif de fixation (10) selon l'une ou l'autre de la revendication 4 ou la revendication 5, dans lequel la couche d'adhésif (35) lie la seconde section d'extrémité droite (32) du niveau d'ancrage à la seconde section d'extrémité droite (42) du niveau d'extension.

7. Dispositif de fixation (10) selon l'une quelconque des revendications 4 à 6, dans lequel la couche d'adhésif (35) lie la première section d'extrémité gauche (31) du niveau d'ancrage à la première section d'extrémité gauche (41) du niveau d'extension.

8. Dispositif de fixation (10) selon l'une quelconque des revendications précédentes, dans lequel le niveau d'extension (40) comprend en outre une queue d'ancrage (48) filant depuis sa première section d'extrémité gauche (41) pour la liaison à la surface d'ancrage (26).

9. Dispositif de fixation (10) selon l'une quelconque des revendications précédentes, dans lequel le niveau de fixation (50) comprend un substrat (54) et une couche d'adhésif (55) sur le côté du substrat (54) adjacent au niveau d'ancrage.

10. Dispositif de fixation (10) selon l'une quelconque des revendications précédentes, comprenant en outre un lève-doigt (56) lié à la première section d'extrémité gauche (51) du niveau de fixation (50).

11. Dispositif de fixation (10) selon l'une quelconque des revendications précédentes, comprenant en outre un ruban cible (70) positionné entre le niveau d'extension (40) et le niveau de fixation (50), dans lequel le ruban cible (70) est de préférence lié de manière amovible au niveau de fixation (50) et porté avec celui-ci au cours du déploiement de la fixation.

12. Dispositif de fixation (10) selon l'une quelconque des revendications 1 à 10, comprenant en outre une pièce à crochet (57) liée au côté du niveau de fixation (50) adjacent au niveau d'ancrage.

13. Couche (20) comprenant un châssis (21) et au moins un dispositif de fixation (10) selon l'une quelconque des revendications précédentes, le châssis (21) comprenant une surface d'ancrage (26) et une surface de fixation (27), la section d'ancrage (33) du niveau d'ancrage (30) étant liée à la surface d'ancrage (26).
